Europäisches Patentamt

European Patent Office

Office européen des brevets

(19)

(11) Veröffentlichungsnummer: **0 081 721**
**B1**

(12) **EUROPÄISCHE PATENTSCHRIFT**

(45) Veröffentlichungstag der Patentschrift:
14.01.87

(51) Int. Cl.⁴: **A 61 K 7/00,** A 61 K 7/32,
A 61 K 7/40

(21) Anmeldenummer: **82110940.2**

(22) Anmeldetag: **26.11.82**

(54) **Kosmetischer, insbesondere desodorierender, Stift.**

(30) Priorität: 03.12.81 DE 3147777

(43) Veröffentlichungstag der Anmeldung:
22.06.83 Patentblatt 83/25

(45) Bekanntmachung des Hinweises auf die Patenterteilung:
14.01.87 Patentblatt 87/3

(84) Benannte Vertragsstaaten:
AT BE CH DE FR GB IT LI NL SE

(56) Entgegenhaltungen:
EP-A-0 013 390
EP-A-0 024 365
FR-A-2 132 130
US-A-2 933 433

G. A. NOWAK, die kosmetischen Präparate", 1975,
Seiten 424-425, Verlag Chem. Industrie

(73) Patentinhaber: Beiersdorf Aktiengesellschaft,
Unnastrasse 48, D-2000 Hamburg 20 (DE)

(72) Erfinder: Fleck, Wolfgang, Dipl.- Chem., Holtwisch
6, D-2000 Hamburg 54 (DE)
Erfinder: Hoppe, Udo, Dipl.- Chem., Lottbeker
Weg 7, D-2000 Hamburg 65 (DE)

## Beschreibung

Die Erfindung betrifft einen kosmetischen Stift zur Anwendung auf der Haut, insbesondere einen desodorierend wirkenden Stift hoher Formstabilität.

Kosmetik-Stifte stellen bedingt durch ihre Kompaktheit und bequeme Handhabbarkeit unter den verschiedenen Formen kosmetischer Präparate unterschiedlicher Art zur Anwendung auf der Haut eine von vielen Verbrauchern bevorzugte, besonders beliebte Form dar. Dies gilt vor allem für desodorierend wirkende Präparate. Das Produkt kann nach dem Auftragen weder von der Hautoberfläche abrinnen (wie bei der Anwendung von flüssigen kosmetischen Zubereitungen) noch die Kleidung beschmutzen oder auf ihr Flecke erzeugen (wie bei der Anwendung von Cremes). Zudem können Stifte, die bekanntlich eingeschlossen in mit einer Schraub- oder Schiebevorrichtung versehenen luftdicht abschließenden Behältern vertrieben werden, im Unterschied zu vielen anderen kosmetischen Anwendungsformen risikolos in Hand und Badetaschen aufbewahrt und mitgeführt werden.

Neben Stiften, die durch Verpressen eines Gemisches aus pulverförmigen Substanzen unter Druck- oder Schlageinwirkung hergestellt werden, den sog. Puderstiften sowie Stiften auf der Grundlage von Fetten und natürlichen oder synthetischen Wachsen nach Art der Lippenstifte, haben unter den kosmetischen Präparaten vor allem solche Stifte eine große praktische Bedeutung erlangt, die aus einem gelartigen Gerüst von Alkali- und/oder Ammoniumsalzen von höheren aliphatischen Carbonsäuren (Fettsäuren) in einem wässrigen, alkoholischen oder wässrig-alkoholischen Medium bestehen. Unter den Alkalisalzen von Fettsäuren (Seifen) wird als Gelbinder das Natriumstearat bevorzugt, das in der Lage ist, mit hohen Anteilen Alkohol hinreichend feste, formbeständige Gele zu bilden. Hierbei können als Alkohole sowohl primäre aliphatische Alkohole, insbesondere Äthanol und Isopropanol, als auch mehrwertige Alkohole wie Glycerin und Propylenglykol, sowie weiterhin Ätheralkohole wie Polyäthylenglykole eingesetzt werden.

Während Puderstifte beim Aufstreichen auf die Haut häufig einen nur unzureichenden Abrieb zeigen und Stifte auf Fett- oder Wachsbasis, abgesehen von ihrer gegenüber den Seifengelstiften fehlenden Transparenz, bei ihrer Anwendung auf der Haut ein unangenehm fettiges Gefühl erzeugen, haben Stifte auf Seifengelbasis neben dem Vorteil der leichten Auftrag- und Verstreichbarkeit und einer häufig erfrischenden, kühlenden Wirkung den Nachteil, daß derartige Stifte nicht in allen Fällen die erwünschte hohe Form-und Dimensionsbeständigkeit aufweisen. Solche Stifte neigen dann durch Verdunsten von Alkoholanteilen zu Schrumpf- und Schwunderscheinungen.

Um Stifte auf der Basis eines gelartigen Gerüsts von Alkalisalzen von hoheren aliphatischen Carbonsäuren (Fettsäuren) als desodorierende Stifte verwenden zu können, müssen diese als Zusatz eine geringe Menge solcher bekannter Substanzen enthalten, die entweder die Abgabe von Körperschweiß an die Hautoberfläche durch Kontraktion der örtlichen Blutgefäße zu vermindern vermögen (Antitranspirantien), z.B. bestimmte Aluminium- oder Zirkoniumsalze, oder die den Teil der bakteriellen Hautflora angreifen und abtöten, der die Zersetzung des Schweißes bewirkt (Deodorantien), z.B. Substanzen mit antibakterieller Wirkung wie 2,4,4'-trichlor-2'-hydroxydi phenyläther.

Aufgabe der Erfindung war die Entwicklung eines Kosmetik-Stiftes mit desodorierender Wirkung auf der Basis eines gelartigen Gerüsts von Alkali- und/oder Ammoniumsalzen höherer aliphatischer Carbonsäuren, der nach dem Auftragen auf die Haut eine desodorierende Wirkung entfaltet, ohne daß dem Stift bei seiner Herstellung eine antibakteriell wirkende Substanz zugesetzt wurde. Die bisher bekannten Stifte dieses Typs für die als Gerüstsubstanz Stearate, insbesondere Natriumstearat, verwendet werden, weisen als solche keine desodorierende Wirkung auf, zur Herstellung eines desodorierenden Kosmetik-Stiftes muß vielmehr stets ein Deomittel und/oder ein Antitranspirationsmittel zugesetzt werden. Für derartige Stifte gemäß dem Stand der Technik nennt beispielsweise Nowak eine Basis-Formel 96 - 92 % Alkohol und 4 - 8 % Natriumstearat, der die Deomittel und Antitranspirationsmittel und ggf. weitere Kosmetikstoffe zugesetzt werden. Dabei kann das Natriumstearat auch in situ durch Verseifung von Stearinsäure mit Natriumhydroxid hergestellt werden (G.A. Nowak: "Die kosmetischen Präparate", 2. Auflage (1975), Seite 424 - 425, Verlag für Chemische Industrie, Augsburg).

Eine weitere Aufgabe der Erfindung bestand darin, ein Produkt nämlich die Wollwachssäuren, einer nutzbringenden Verwendung zuzuführen, das als Nebenprodukt bei der Verseifung des Wollwachses zur Gewinnung der als Emulgator vom typ W/O sehr begehrten Wollwachsalkohole in erheblicher Menge anfällt und für das man bisher trotz großer Bemühungen keine rechte Verwendung gefunden hat.

Es wurde überraschenderweise gefunden und darin liegt die Lösung der Aufgabe gemäß der Erfindung, daß man zu ausgezeichneten Kosmetik-Stiften von hoher Formstabilität mit desodorierender Wirkung ohne Zusatz eines antibakteriell wirkenden Mittels dann gelangt, wenn man einen desodorierenden Kosmetik-Stift auf der Basis eines überwiegend aus Alkoholen sowie Alkali- und/oder Ammoniumsalzen höherer aliphatischer Carbonsäuren als formgebender Gerüstsubstanz bestehenden wässrig/alkoholischen Gels, ggf. unter Zusatz weiterer Kosmetikstoffe, in an sich bekannter Weise herstellt, indem man anstelle der Alkali-und/oder Ammoniumsalze höherer aliphatischer Carbonsäuren als formgebende Gerüstubstanz Alkali- und/oder Ammoniumsalze von Wollwachssäuren oder deren Salze mit Triäthanolamin einsetzt.

Der in den Wollwachssäuren vorhandene Gehalt an iso-$C_{14}$- und iso-$C_{16}$-Carbonsäuren mit bekannter bakterizider Wirkung bewirkt, daß diese Stifte eine gute desodorierende Wirkung aufweisen ohne des Zusatzes eines speziellen Deowirkstoffes zu bedürfen.

Gegenstand der Erfindung ist somit ein kosmetischer, insbesondere desodorierender, Stift auf der Basis eines wässrig-alkoholischen Gels von Alkaii- und/oder Ammoniumsalzen höherer aliphatischer Carbonsäuren als formgebende Gerüstsubstanz, der dadurch gekennzeichnet ist, daß dieser neben vorzugsweise hohen Anteilen an ein- oder mehrwertigen Alkoholen, einer kleinen Menge Wasser sowie geringen Anyeilen üblicher kosmetischer Zusatzstoffe als formgebende Gerüststubstanz (Gelbildner) 1 bis 15 Gew.-% vorzugsweise 4 bis 10 Gew.-%, jewelis bezogen auf das Gesamtgewicht des Stiftes, Alkali- und/oder Ammoniumsalze von Wollwachssäuren oder deren Salze mit Triäthanolamin enthält.

Vorzugsweise kann die formgebende Gerüstsubstanz aus dem Natriumsalz der Wollwachssäuren bestehen.

Die für die Stiftherstellung benötigten Wollwachssäuren werden durch Verseifung von Wollwachs (Wollfett) mit äthanolischem Alkali nach üblichem Verfahren gewonnen. Nach Abtrennung der Wollwachsalkohole durch Extraktion mit einem organischen Lösungsmittel werden die Metallseifen angesäuert, wodurch die freien Säuren (Wollwachssäuren) in Form einer gelbbräunlichen klebrigen Masse von wachsartiger Konsistenz erhalten werden. Diese Rohsäuren werden zur Reinigung und Abtrennung unerwünschter Substanzen einer Destillation in Hochvakuum oder einer Molekulardestillation unterworfen, wobei reine Wollwachssäuren als weißlich-gelbes Produkt von angenehmem Geruch anfallen, das als Ausgangsprodukt zur Herstellung der erfindungsgemäß als formbildende Gerüstsubstanz verwendeten Alkalibzw. Ammoniumsalze oder des Umsetzungsprodukts mit Triäthanolamin dient. Aus dem Wollwachssäureanteil des verseiften Wollwachses konnten 36 verschiedene Säuren isoliert werden, die sämtlich der aliphatischen Reihe angehören und die über ihre Methylester in vier Gruppen aufgeteilt werden können (n-Carbonsäuren, Oxycarbonsäuren, iso-Carbonsäuren und ante-Isocarbonsäuren).

Da in den gereinigten Wollwachssäuren die bakterizide Wirkung auf den Gehalt an bestimmten Verbindungen aus der Reihe der iso-Carbonsäuren beruht, kann gemäß einer bevorzugten Ausführungsart der Erfindung für die Salzbildung anstelle der normalen gereinigten Wollwachssäuren eine über die Methylester der Wollwachssäuren isolierte Fraktion der Wollwachssäuren eingesetzt werden, die einen stark angereicherten Gehalt an Iso-Säuren aufweist.

Die Herstellung der Kosmetik-Stifte gemäß der Erfindung erfolgt zweckmäßigerweise in der Art, daß die Wollwachssäuren im Gemisch mit den benötigten Alkoholen und gegenbenenfalls weiteren Zusätzen mit Alkali oder Ammoniak verseift werden. Anstatt die Verseifung der Wollwachssäuren in Gegenwart der benötigten Alkohole an Ort und Stelle vorzunehmen, können auch die bereits vorgebildeten Seifen in kleinen Anteilen dem Gemisch aus Alkoholen und den weiteren Bestandteilen zugesetzt werden. Nach Ausbildung einer homogenen Masse wird diese nach eventuellem Zusatz von Duft- und/oder Farbstoffen im warmen Zustand in entsprechende Formen oder Behälter eingegossen. Art und Reihenfoige, in der die Bestandteile gemischt oder dem Gemisch zugesetzt werden, sind nicht kritisch.

Zur Bildung der Salze von Wollwachssäuren kann an Stelle von Alkali oder Ammoniak auch Triäthanolamin eingesetzt werden.

Dabei können als Alkohole sowohl einwertige Alkohole (wie Äthanol) als auch mehrwertige Alkohole (wie Propylenglykol, Glycerin) oder deren Mischungen eingesetzt werden. Zwecks Erzielung einer verstärkten desodorierenden Wirkung oder falls besondere Einsatzbedingungen dieses erfordern, können den Stiften gemäß der Erfindung zusätzlich 0,1 bis 0,2 Gew.-%, bezogen auf die Gesamt-Zusammensetzung, eines antibakteriell wirksamen Mittels, wie 2,4,4'-trichlor-2'-hydroxydiphenyläther oder Undecylensäurepolydiäthanolamid, zugesetzt werden.

Zur Herstellung eines Antitranspirationsmittels können dem Stift 5,0 bis 10,0 Gew.-%, bezogen auf die Gesamt-Zusammensetzung, an bestimmten, im wässrig-alkoholischen alkalischen Milieu beständigen Aluminiumverbindungen, wie Natriumaluminiumchlorhydroxylactat ("Chloracel®") oder Aluminiumhydroxychlorid-Propylenglykol-Komplex-Verbindungen ("Rehydrol ASC"®), zugesetzt werden; zur Herstellung eines Stiftes mit Insektenabweisenden Eigenschaften bis zu etwa 10 Gew.-% eines Wirkstoffs mit Insect-Repellent-Wirkung, beispielsweise 2-Äthylhexandiol-1,3 und insbesondere 3-(N-n-butyl-N-acetyl)-amino-propionsäure-äthylester.

Anstelle eines der vorstehend genannten Wirkstoffe kann - falls dies gewünscht wird - auch ein Gemisch aus zwei dieser Substanzen eingesetzt werden.

Zur Erhöhung der Formstabilität des Stiftes und zur Erzielung eines besseren Abriebs können den Massen für die Herstellung des Stiftes außerdem geringe Mengen (2 bis 5 Gew.-%, vorzugsweise etwa 3 Gew.-%, bezogen auf die Gesamtzusammensetzung) an Emulgatoren zugesetzt werden. Hierfür haben sich nichtionogene Typen, wie Sorbitanester und Sorbitanester-Äthylenoxid-Verbindungen (z.B. Sorbitanmonostearat und Polyoxyäthylensorbitanmonostearat) als auch Polyoxyäthylen-Fettalkoholäther und langkettige höhermolekulare wachsartige Polyglykoläther als besonders geeignet erwiesen.

Zusätzlich zu den genannten Bestandteilen können den Gelstift. Zusammensetzungen kleine Mengen Parfüm, Farbstoffe, Feuchthaltemittel, Antioxidantien, Komplexbildner oder andere übliche kosmetische Grundstoffe, wie 2-Octyldodecanol, Decyloleat oder Ölsäureoleylester, beigemischt werden.

Zur Parfümierung der Stifte sind solche Substanzen geeignet, die stabil sind, beständig gegen Alkali und die Haut nicht reizen. Vorzugsweise soll der pH-Wert der Stifte etwa 8,5 bis 9,8 betragen. Zur Neutralisation eines Überschusses an Alkali kann Weinsäure oder Zitronensäure in der erforderlichen Menge zugesetzt werden.

Zum Nachweis der überlegenen, desodorierenden Wirkung der erfindungsgemäßen Kosmetik-Stifte ohne Zusatz eines Deo-Wirkstoffs gegenüber den bekannten Stiften auf Seifengelbasis mit Deo-Wirkstoff-Zusatz wurden zwei Stifte hergestellt, von denen bei ansonsten völlig identischer Zusammensetzung der eine (Stift 1) 6% Wollwachssäuren und der andere (Stift 2) statt dessen 6% Stearinsäure sowie 1% Undecylensäurepolydiäthanolamid als Deo-Wirkstoff enthielt. In beiden Fällen war die Säure mit der gleichen Menge Natronlauge durch Verseifung in das entsprechende Natriumsalz überführt worden (formgebende Gerüstsubstanz).

Die desodorierende Wirkung des Stiftes gemäß der Erfindung (Stift 1) wurde in einem Plattendiffusionstest gegenüber einem Seifengelstift bekannter Art (Stift 2) mit einem Zusatz von Undecylensäure-Polydiäthanolamid, einer bekannten antimykotisch und bakteriostatisch wirksamen Substanz (vgl. H. Fey:"Wörterbuch der Kosmetik", Wiss. Verlags Ges. Stuttgart 1974, Seite 414), im Verhalten zu bestimmten Bakterienkulturen getestet.

Die Ergebnisse dieses Vergleichsversuchs sind in der folgenden Tabelle zusammengefaßt (Zahlenangaben = Hemmzone in mm):

| Stamm | Staph. aureus | Staph. albus | Corynebact. | Ps. aeruginose |
|---|---|---|---|---|
| Stift 1 | 5 - 6 | 5 - 6 | 10 | 5 |
| Stift 2 | 4 - 5 | 4 - 5 | 9 - 10 | 3 - 5 |

Da eine vergrößerte Hemmzone einer besseren desodorierenden Wirkung entspricht, ergibt sich aus dieser Tabelle die überlegene Wirkung des Stiftes gemäß der Erfindung ohne zusätzlichen Deo-Wirkstoff gegenüber einem Stift bekannter Zusammensetzung mit Zusatz von 1 % Deo-Wirkstoff.

Die Erfindung wird anschließend anhand von Beispielen näher erläutert:

**Beispiel 1**

| Deodorant-Stift | Gew.-Teile |
|---|---|
| Wollwachssäuren | 7,0 |
| Natriumhydroxid | 1,2 |
| Propylenglykol | 35,0 |
| Glycerin | 50,0 |
| Antioxydans (2,6-Di-tert.butyl-4-methyl-phenol "Ionol®") | 0,1 |
| Wasser, entmineralisiert | 5,2 |
| Parfüm | 1,5 |

In einem Kessel mit Rückflußkühler wurden Wollwachssäuren, Propylenglykol, Glycerin und das Antioxydationsmittel bei erhöhter Temperatur (etwa 60° C) miteinander vermischt, alsdann eine Lösung des Natriumhydroxids in Wasser hinzugefügt und anschließend eine halbe Stunde lang bei 75° C verseift. Nach dem Abkühlen wurde das Parfüm bei etwa 60° unter Rühren der Masse zugefügt und die flüssige Masse daraufhin bei dieser Temperatur in die entsprechenden Formen eingegossen und erkalten gelassen. Es wurden Stifte guter Formbeständigkeit mit ausgezeichneter desodorierender Wirkung nach dem Auftragen auf die Haut erhalten.

**Beispiel 2**

| Deodorant-Stift | Gew.-Teile |
|---|---|
| Na-salz der Wollwachssäuren | 8,8 |
| Äthanol | 58,0 |
| Propylenglykol | 26,0 |
| Emulgator (Polyoxyäthylen-Cetyläther, | |
| "Brij B 58" (Atlas-Chemie, Essen) | 3,0 |
| Antioxydans ("Ionol") | 0,1 |
| Deomittel (2,4,4'-Trichlor-2'-hydroxy- | |
| diphenyläther) | 0,2 |
| Wasser (entmineralisiert) | 2,4 |
| Parfüm | 1,5 |

Eine Mischung aus dem Natriumsalz der Wollwachssäuren, Äthanol, Propylenglykol, Emulgator, Antioxydans, Deomittel und Wasser wurde in einem Kessel mit Rückflußkühler 45 Minuten lang unter Rückfluß auf 80°C erwärmt. Die Parfümzugabe erfolgte unter kräftigem Rühren bei 65°C. Anschließend wurde die flüssige Masse in entsprechende Formen gegossen und darin erkalten gelassen.

**Beispiel 3**

| Antitranspirant-Stift | Gew.-Teile |
|---|---|
| Wollwachssäuren | 6,0 |
| Natriumhydroxid | 1,1 |
| Propylenglykol | 30,0 |
| Glycerin | 22,0 |
| Natrium-Aluminium-Chlorhydroxylactat | |
| ("Chloracel" in Form einer 60 %igen | |
| wässr. Lösung) | 20,0 |
| Emulgator (Polyoxyäthylen-Cetylester, | |
| "Brij B 58" (Atlas-Chemie, Essen | 3,0 |
| Wasser (entmineralisiert) | 15,9 |
| Parfüm | 2,0 |

Propylenglykol, Glycerin, Emulgator und Wasser) wurde bei erhöhter Temperatur mit 1,1 g Natriumhydroxid und weiteren 2 g Wasser (entmin.) 30 Minuten lang bei einer Temperatur von 75°C verseift. Die 60%ige "Chloracel®"-Lösung wurde auf 70° erwärmt und unter Rühren vorsichtig hinzugegeben. Nach Parfümierung bei etwa 65°C wurde die flüssige Masse bei etwa 60°C in die verbreiteten Formen für die Stiftherstellung eingegossen und erstarren gelassen.

**Beispiel 4**

| Insekt-Repellent-Stift | Gew.-Teile |
|---|---|
| Na-Salz der Wollwachssäuren | 8,8 |
| Emulgator (langk. höhermol. wachsartiger | |
| Polyglykoläther, "Cremophor A 25", BASF) | 3,0 |
| 2-Octyldodecanol | 22,0 |
| Äthanol | 42,0 |
| Insektenabwehrmittel (3-(N-n-butyl-N- | |
| acetyl)-aminopropionssäureäthylester) | 10,0 |
| Wasser, entmineralisiert | 11,7 |
| Parfüm | 2,5 |

In einem Kessel mit Rückflußkühlung wurde eine Mischung der oben angegebenen Bestandteile (Natriumsalz der Wollwachssäuren, Wasser, Äthanol, Emulgator und 2-Octyldodecanol) 45 Minuten lang unter Rückfluß auf 80°C erwärmt. Bei 70°C erfolgte dann unter Rühren die anteilsweise Zugabe des Insektenabwehrmittels. Das Parfüm wurde bei einer Temperatur von 65°C zugegeben und die flüssige Masse alsdann bei 60°C in die entsprechenden Formen eingegossen.

Es wurden in allen Fällen kosmetische Stifte mit guter Transparenz und hoher Formstabilität erhalten, die beim Auftragen auf die Haut einen leichten Abrieb zeigten.

**Patentansprüche**

1. Desodorierender Kosmetik-Stift auf der Basis eines überwiegend aus Alkoholen sowie Alkali- und/oder Ammoniumsalzen höherer aliphatischer Carbonsäuren als formgebender Gerüstsubstanz bestehenden alkoholischwässrigen Gels, gegebenenfalls unter Zusatz weiterer Kosmetikstoffe, dadurch gekennzeichnet, daß er aliphatischer Carbonsäuren als formgebende Gerüstsubstanz Alkali- und/oder Ammoniumsalze von Wollwachssäuren oder deren Salze mit Triäthanolamin enthält.

2. Desodorierender Kosmetik-Stift nach Anspruch 1, dadurch gekennzeichnet, daß die formgebende Gerüstsubstanz aus dem Natriumsalz der Wollwachssäuren besteht.

3. Desodorierender Kosmetik-Stift nach Anspruch 1, dadurch gekennzeichnet, daß dieser zusätzlich ein Deomittel, ein Antitranspirationsmittel, ein Insekten abwehrendes Mittel oder ein Gemisch aus zwei dieser Substanzen enthält.

## Claims

1. Deodorizing cosmetic stick based on an alcoholic-aqueous gel consisting chiefly of alcohols and alkali metal and/or ammonium salts of higher aliphatic carboxylic acids as the matrix substance providing shape, if appropriate with the addition of other cosmetic substances, characterized in that, instead of the alkali metal and/or ammonium salts of higher aliphatic carboxylic acids as the matrix substance providing shape, it contains alkali metal and/or ammonium salts of wool wax acids or salts thereof with triethanolamine.

2. Deodorizing cosmetic stick according to Claim 1, characterized in that the matrix substance providing shape consists of the sodium salt of wool wax acids.

3. Deodorizing cosmetic stick according to Claim 1, characterized in that this additionally contains a deodorant, an antiperspirant, an insect repellent or a mixture of two of these substances.

## Revendications

1.- Bâton de cosmétique déodorant à base d'un gel alcoolique/aqueux constitué principalement par des alcools ainsi que des sels alcalins et/ou ammoniques d'acides carboxyliques aliphatiques supérieurs comme substance du squelette donnant la forme, éventuellement avec addition d'autres agents cosmétiques, caractérisé en ce qu'il contient, au lieu des sels alcalins et/ou ammoniques d'acides carboxyliques aliphatiques supérieurs comme substance du squelette donnant la forme, des sels alcalins et/ou ammoniques d'acides de la lanoline ou les sels de ceux-ci avec la triéthanolamine.

2.- Bâton de cosmétique déodorant suivant la revendication 1, caractérisé en ce que la substance du squelette donnant la forme consiste en le sel de sodium des acides de la lanoline.

3.- Bâton de cosmétique déodorant suivant la revendication 1, caractérisé en ce qu'il contient, en outre, un agent déodorant, un agent antiperspirant, un agent insectifuge ou un mélange de deux de ces substances.